Europäisches Patentamt

European Patent Office

Office européen des brevets

(11) Numéro de publication: **0 207 829**
A1

## (12) DEMANDE DE BREVET EUROPEEN

(21) Numéro de dépôt: 86401176.2

(22) Date de dépôt: 03.06.86

(51) Int. Cl.⁴: **A 61 B 10/00**
**A 61 B 17/32**

(30) Priorité: 04.06.85 FR 8508386

(43) Date de publication de la demande:
07.01.87 Bulletin 87/2

(84) Etats contractants désignés:
DE FR GB IT

(71) Demandeur: Schintgen, Jean-Marie
45 Avenue Victor Hugo
F-75116 Paris(FR)

(71) Demandeur: Zeitoun, Bruno
23 rue Daubenton
F-75005 Paris(FR)

(72) Inventeur: Schintgen, Jean-Marie
45 Avenue Victor Hugo
F-75116 Paris(FR)

(72) Inventeur: Zeitoun, Bruno
23 rue Daubenton
F-75005 Paris(FR)

(74) Mandataire: Dawidowicz, Armand
30, Boulevard du Château
F-92200 Neuilly(FR)

(54) Pince à biopsie à aiguillon rétractable.

(57) L'invention concerne une pince à biopsie du type comprenant deux cuillères ou mords articulés chacun sur une biellette de commande à une extrémité d'une gaine, lesdites biellettes étant articulées sur une pièce solidaire d'un câble coulissant dans ladite gaine sous la commande d'une poignée coulissant dans l'extrémité opposée ladite gaine, un aiguillon de fixation étant logé entre les cuillères ou mords.

La pince à biopsie selon l'invention est caractérisée par le fait que lesdites biellettes (9,10) sont articulées sur ledit aiguillon (5) et les mords (3,4) sont articulés sur un axe fixe (7) sur lequel coulisse une lumière longitudinale (8) dudit aiguillon (5).

Application aux pinces à biopsie.

Pince à biopsie à aiguillon rétractable.

L'invention concerne une pince à biopsie du type comprenant deux cuillères ou mords articulés chacun sur une biellette de commande à une extrémité d'une gaine, lesdites biellettes étant articulées sur une pièce solidaire d'un câble coulissant dans ladite gaine sous la commande d'une poignée coulissant dans l'extrémité opposée de ladite gaine, un aiguillon de fixation étant logé entre les cuillères ou mords.

Dans les pinces à biopsie connues du type précédent, l'aiguillon de fixation, qui sert à fixer la pince à l'endroit où l'on souhaite effectuer le prélèvement, est monté fixe entre les deux mords. Cette disposition a l'inconvénient d'occuper à demeure un volume relativement important qui est perdu pour le prélèvement effectué entre les deux mords.

En outre, dans ces pinces à biopsie connues, les mords sont articulés l'un sur l'autre et l'extrémité de chacun d'entre eux opposée à leur portion pinçante est articulée sur une biellette, les deux biellettes étant elles-mêmes articulées sur une bielle coulissant dans l'extrémité correspondante de la gaine et fixée à l'extrémité adjacente du câble de commande. Cette disposition usuelle présente l'inconvénient que le parallélogramme articulé formé par les deux mords et leurs biellettes de commande, articulé en un point de la bielle, peut basculer autour de ce point, ce qui est très gênant pour la précision et l'efficacité du prélèvement. En outre, le montage de cet ensemble, de très petite dimension, est très délicat.

La présente invention a pour but de pallier ces inconvénients des pinces à biopsie connues grâce à une nouvelle pince de construction simplifiée dans laquelle l'aiguillon est rétracté au cours de la fermeture de la pince et les mords ne peuvent pas basculer au cours de l'ouverture ou de la fermeture de la pince.

A cet effet, la pince à biopsie selon l'invention est caractérisée par le fait que lesdites biellettes sont articulées sur ledit aiguillon et les mords sont articulés sur un axe fixe sur lequel coulisse une lumière longitudinale dudit aiguillon.

De préférence, afin d'améliorer l'arrimage de l'aiguillon dans la muqueuse au cours de la fermeture des pinces, l'invention prévoit que l'aiguillon de fixation est muni de crans latéraux.

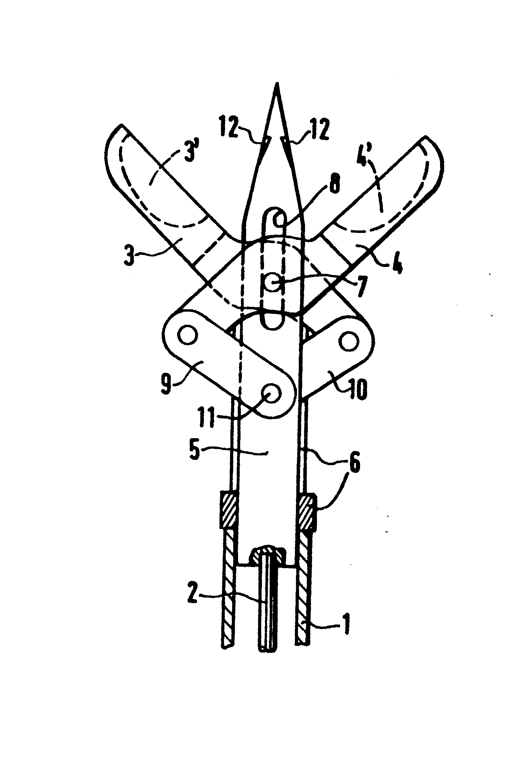

L'invention sera bien comprise à la lecture de la description suivante faite en se référant au dessin annexé dont la figure unique est une vue schématique de l'extrémité pinçante de la pince à biopsie selon un exemple de réalisation de l'invention.

La pince à biopsie comprend une gaine 1 dans laquelle coulisse un câble 2 dont l'extrémité opposée à l'extrémité pinçante (représentée au dessin) est commandée par une poignée (non représentée). Le câble 2, au cours de son coulissement dans la gaine 1 sous la commande de la poignée, provoque le pivotement l'un par rapport à l'autre de deux cuillères ou mords 3 et 4, comportant chacun un évidement 3', 4' respectivement, le volume ménagé par les évidements 3', 4', lorsque la pince constituée par les mords 3 et 4,               est fermée, formant le volume de prélèvement de la pince à biopsie. Un aiguillon 5 est destiné à fixer la pince pour le prélèvement.

Conformément à la forme de réalisation décrite de l'invention, le câble 2 est fixé à l'extrémité postérieure de l'aiguillon 5 qui est coulissant dans une chape 6 fixée sur la gaine 1. Les mords 3 et 4 sont articulés, en une partie intermédiaire de leur longueur, sur un rivet 7 solidaire de la chape 6 sur lequel coulisse une lumière longitudinale 8 de l'aiguillon 5. A leur extrémité opposée aux évidements 3', 4' respectivement, les mords 3 et 4 sont articulés chacun sur une biellette 9, 10 respectivement. Les biellettes 9 et 10, à leur extrémité opposée aux mords 3 et 4, sont articulés sur un axe commun 11, formé par un rivet, fixé sur l'aiguillon 5.

Le coulissement du câble 2 entraîne l'aiguillon 5, et l'axe 11 qui en est solidaire, en un coulissement égal. Les mords 3 et 4 pivotent autour de l'axe fixe 7 sur lequel glisse la lumière 8. Par traction sur le câble 2, la pince constituée par les mords 3 et 4 se referme pendant que l'aiguillon 5 s'efface presque complètement dans le volume formé par les évidements 3', 4', qui est ainsi maximal. La pince 3,4, guidée en deux points 7, 11, ne peut pas basculer de part et d'autre de l'axe du câble 2, comme c'était le cas avec les pinces à biopsie connues.

Le montage de l'ensemble qui vient d'être décrit est plus facile et plus rapide que celui des pinces à biopsie connues. En outre, par rapport à une pince à biopsie à aiguillon connue, la pince selon l'invention, dans laquelle l'aiguillon et la bielle sont une pièce unique, comporte une pièce de moins et économise des opérations de montage, de sertissage et de perçage. La pince à biopsie selon l'invention est donc plus économique que les pinces à biopsie connues.

Selon une forme de réalisation préférée de l'invention, on prévoit que la pointe de l'aiguillon 5 est munie de crans latéraux 12. Au cours du retrait de l'aiguillon 5 provoquant la fermeture de la pince 3,4 et l'enfoncement des bords tranchants des mords dans une muqueuse pour effectuer la biopsie, ces crans 12 s'opposent à la sortie de l'aiguillon 5 à l'extérieur de la muqueuse, ce qui favorise l'arrimage de la pince dans la muqueuse et améliore le prélèvement, en particulier dans le cas de muqueuses résistantes.

0207829

Revendications.

1. - Pince à biopsie du type comprenant deux cuillères ou mords articulés chacun sur une biellette de commande à une extrémité d'une gaine, lesdites biellettes étant articulées sur une pièce solidaire d'un câble coulissant dans ladite gaine sous la commande d'une poignée coulissant dans l'extrémité opposée de ladite gaine, un aiguillon de fixation étant logé entre les cuillères ou mords, caractérisée par le fait que lesdites biellettes (9,10) sont articulées sur ledit aiguillon (5) et les mords (3,4) sont articulés sur un axe fixe (7) sur lequel coulisse une lumière longitudinale (8) dudit aiguillon (5).

2. - Pince à biopsie selon la revendication 1, caractérisée par le fait que l'aiguillon de fixation (5) est muni de crans latéraux (12).

Office européen
des brevets

**RAPPORT DE RECHERCHE EUROPEENNE**

**0207829**

Numéro de la demande

EP  86 40 1176

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int Cl.4) |
|---|---|---|---|
| Y | DE-A-3 441 036 (HAYASHI et al.) * Page 6, lignes 5-10; figure 2 * | 1 | A 61 B 10/00 A 61 B 17/32 |
| Y | US-A-3 895 636 (SCHMIDT) * Colonne 2, ligne 65 - colonne 3, ligne 1; figures * | 1 | |
| A | FR-A-2 253 490 (HALPERN et al.) * Page 9, lignes 6-10; figures * | 1,2 | |
| A | US-A-3 147 749 (MARSH) * Colonne 3, ligne 73 - colonne 4, ligne 4; figures * | 1,2 | |
| A | DE-A-2 506 471 (KOMIYA) * Figure 1; page 3, lignes 4-15 * | 1 | |

| | | |
|---|---|---|
| | | **DOMAINES TECHNIQUES RECHERCHES (Int. Cl.4)** |
| | | A 61 B |

Le present rapport de recherche a été établi pour toutes les revendications

| Lieu de la recherche | Date d'achèvement de la recherche | Examinateur |
|---|---|---|
| LA HAYE | 12-09-1986 | GLAS J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82